# EUROPÄISCHE PATENTSCHRIFT

(11) **EP 1 033 146 B1**
(45) Veröffentlichungstag und Bekanntmachung des Hinweises auf die Patenterteilung: **17.07.2002**
(21) Anmeldenummer: 00101152.7
(22) Anmeldetag: 21.01.2000
(51) Int. Cl.: A61M 39/12

(54) **Katheterkupplung**
Connection for catheter
Couplement pour cathéter

(30) Priorität: 24.02.1999 DE 29903286 U
(43) Veröffentlichungstag der Anmeldung: 06.09.2000
(73) Patentinhaber: B. BRAUN MELSUNGEN AG, 34212 Melsungen (DE)
(72) Erfinder: Göbel, Udo, 34212 Melsungen-Kirchhof (DE); Otto, Hans-Joachim, 34212 Melsungen (DE); Sippel, Martin, Dr., 34212 Melsungen (DE)
(74) Vertreter: Selting, Günther, Dipl.-Ing.

(56) Entgegenhaltungen:
- GB-A- 2 060 400
- US-A- 4 723 948
- US-A- 5 556 136

## Beschreibung

Die Erfindung betrifft eine Katheterkupplung mit integrierter Zugentlastung zur Ermöglichung des Ankuppeins eines Katheters an eine Spritze oder an eine andere Flüssigkeitsübertragungsvorrichtung.

Bei der Epiduralanästhesie wird ein Katheter durch eine Stahlkanüle hindurch in den Epiduralraum eines Patienten eingeführt. Anschließend wird die Stahlkanüle über das proximale Katheterende hinweg zurückgezogen, während der Katheter an seinem Platz verbleibt. Das proximale Ende des Katheters darf hierbei keine Verdickung oder Anschlußvorrichtung aufweisen, weil hierdurch das Zurückziehen der Stahlkanüle behindert würde. Nachdem die Stahlkanüle entfernt wurde, muß der Katheter jedoch mit einer Katheterkupplung, beispielsweise in Form eines Luer-Lock-Verbinders, versehen werden, damit er an eine flüssigkeitsliefernde Vorrichtung angeschlossen werden kann. Der Katheter besteht aus einem flexiblen Schlauch von geringem Durchmesser. Der Außendurchmesser beträgt in der Regel weniger als 1 mm. Eine Katheterkupplung, die mit dem Katheter verbunden wird, darf den Katheter nicht quetschen, damit das Katheterlumen offen bleibt.

Aus EP 0 415 665 A1 ist eine Katheterkupplung bekannt, bei der der Katheter durch einen ringförmigen elastomeren Stopfen hindurchgesteckt wird, der zwischen zwei Schraubteilen eingeklemmt ist. Durch Festdrehen der Schraubteile wird der Stopfen radial gedehnt und mit Kraft gegen den Katheter gedrückt. Zum Bedienen der Katheterkupplung sind zwei Hände erforderlich, während zugleich der Katheter festgehalten werden muß. Ein geordnetes Festziehen der Katheterkupplung würde somit drei Hände erfordern. Außerdem ist zum Festziehen eine große Kraft notig. Wenn die Katheterkupplung einen Luer-Lock-Anschluß aufweist, der zum Lösen gedreht werden muß, besteht die Gefahr, daß anstelle des Luer-Lock-Anschlusses die Katheterkupplung geöffnet wird, so daß der Katheter herausgleitet.

Eine andere Schlauchkupplung ist in DE 41 29 781 A1 beschrieben. Hierbei enthält die Katheterkupplung ein trompetenförmiges Rohrteil, auf dessen schmaleres Ende der Katheter aufgeschoben wird. Anschließend erfolgt das Festklemmen mit zwei Klemmteilen, die mit einem Bajonettverschluß gespannt werden. Auch hierbei kann durch unbeabsichtigtes Drehen des Bajonettverschlusses ein Lösen erfolgen.

Eine Katheterkupplung mit zwei gelenkig verbundenen Backen ist aus US 4 006 744 bekannt. Hierbei ist einer der Backen mit einem Schlauchstück versehen, durch das der Katheter hindurchgesteckt wird. Der andere Backen wird über das Schlauchstück geklappt und klemmt dieses fest. Der Katheter wird nur durch das radial gepreßte Schlauchstück festgehalten. Dabei besteht die Gefahr des unbeabsichtigten Herausziehens des Katheters.

Der Oberbegriff des Anspruchs 1 geht von diesem Stand der Technik aus.

Der Erfindung liegt die Aufgabe zugrunde, eine Katheterkupplung mit Zugentlastung zu schaffen, die einfach an den Katheter anzulegen ist und eine hohe Haltekraft gewährleistet, ohne den Katheter abzuschnüren.

Die erfindungsgemäße Katheterkupplung weist die Merkmale des Patentanspruchs 1 auf.

Erfindungsgemäß verlaufen die Backen in Längsrichtung des das Katheterende aufnehmenden Schlauchstücks und sie sind an dem einen Ende des Schlauchstücks durch ein Gelenk verbunden, dessen Achse quer zu dem Schlauchstück verläuft. Am gegenüberliegenden Ende sind die Backen im Schließzustand verriegelbar. Die Backen übergreifen das einzuspannende Endstück des Katheters also in Längsrichtung, wodurch ein besonders guter und sicherer Halt gewährleistet ist, weil ein langer Überdeckungsbereich realisiert werden kann. Hinzukommt, daß beim Schließen der Backen die Klemmkraft von einem Ende bis zum anderen fortschreitend zunimmt. Auf diese Weise wird über einen relativ langen Weg eine sichere Klemmung erreicht.

Vorzugsweise sind die in den Backen gebildeten Rinnen, die den Kanal bilden, nicht mit einem gerade durchgehenden Rinnenboden versehen, sondern der Rinnenboden hat Erhöhungen und/oder Vertiefungen, um bei geschlossenen Backen einen schlangenlinienförmigen Verlauf des Schlauchstücks hervorzurufen. Dadurch wird ein hoher Reibwiderstand erzeugt.

Die Katheterkupplung ist sehr leicht zu handhaben. Es ist lediglich erforderlich, die Backen gegeneinander zu drücken, um den Kanal zu schließen. Andererseits besteht nicht die Gefahr, daß die Katheterkupplungen durch Drehbewegungen, wie sie beim Lösen von einem Gegensteckverbinder erforderlich sind, versehentlich aufgehen kann.

Vorzugsweise hat der Kanal mindestens zwei Biegungen, zwischen denen sich ein längslaufender Abschnitt befindet. Hierbei wird der Schlauch nur in den Biegungen verformt, nicht aber in dem geraden Abschnitt. Auf diese Weise wird sichergestellt, daß der Schlauch bei geöffneten Backen einen gestreckten geraden Zustand annimmt, so daß der Katheter leicht in den Schlauch eingeführt werden kann.

Im folgenden werden unter Bezugnahme auf die Zeichnungen Ausführungsbeispiele der Erfindung näher erläutert.

Es zeigen:
- Fig. 1: eine Seitenansicht der Katheterkupplung,
- Fig. 2: eine Stirnansicht aus Richtung des Pfeiles II von Fig. 1,
- Fig. 3: die Katheterkupplung bei geöffneten Backen,
- Fig. 4: in vergrößertem Maßstab eine Draufsicht aus Richtung des Pfeiles IV von Fig. 1,
- Fig. 5: eine ähnliche Darstellung wie Fig. 1, wobei der Schlauch erkennbar ist,
- Fig. 6: einen Längsschnitt durch die geöffneten Backen mit dem dazwischen angeordneten Schlauch und dem darunter dargestellten Katheter,
- Fig. 7: einen Schnitt entlang der Linie VII-VII von Fig. 5,
- Fig. 8: einen Schnitt entlang der Linie VIII-VIII von Fig. 5,
- Fig. 9: ein weiteres Ausführungsbeispiel,
- Fig. 10: den unteren Backen der Katheterkupplung nach Fig. 9 im Langsschnitt,
- Fig. 11: einen Schnitt entlang der Linie XI-XI von Fig. 9 und
- Fig. 12: einen Schnitt entlang der Linie XII-XII von Fig. 9.

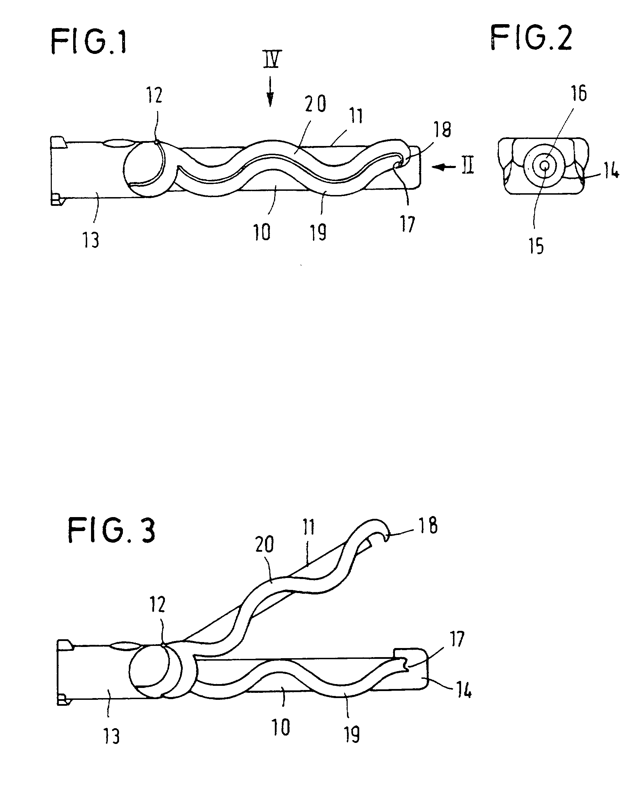

Die dargestellte Katheterkupplung weist zwei langgestreckte, im wesentlichen plattenförmige Backen 10,11 auf, die an einem Ende durch ein Gelenk 12 miteinander verbunden sind. Der Backen 10 bildet einen Basisbacken, der starr mit dem Verbindungsteil 13 verbunden ist und dieses in axialer Richtung verlängert, während der Backen 11 gemäß Fig. 3 aufgeklappt werden kann.

Der Backen 10 hat an seinem dem Gelenk 12 abgewandten Ende eine Stirnwand 14 mit einer durchgehenden Öffnung 15 zum Hineinstecken eines Katheters. Um die Öffnung 15 herum ist ein Einführtrichter 16 ausgebildet.

Die Backen 10,11 haben an dem dem Gelenk 12 abgewandten Ende zusammengreifende Verriegelungselemente 17,18. Das Verriegelungselement 17 besteht aus einer Ausnehmung an dem Backen 10 und das Verriegelungselement 18 besteht aus einem elastischen Haken, der bei geschlossenen Backen in der Ausnehmung 17 einrastet.

Der Backen 10 hat an seinen beiden Seiten schlangenlinienförmige Ränder 19 und der Backen 11 hat hierzu passende schlangenlinienförmige Ränder 20. Im Schließzustand gemäß Fig. 1 liegen die Ränder 19 und 20 passend aneinander, während die Verriegelungselemente 17,18, die an den Enden der Ränder vorgesehen sind, zusammengreifen.

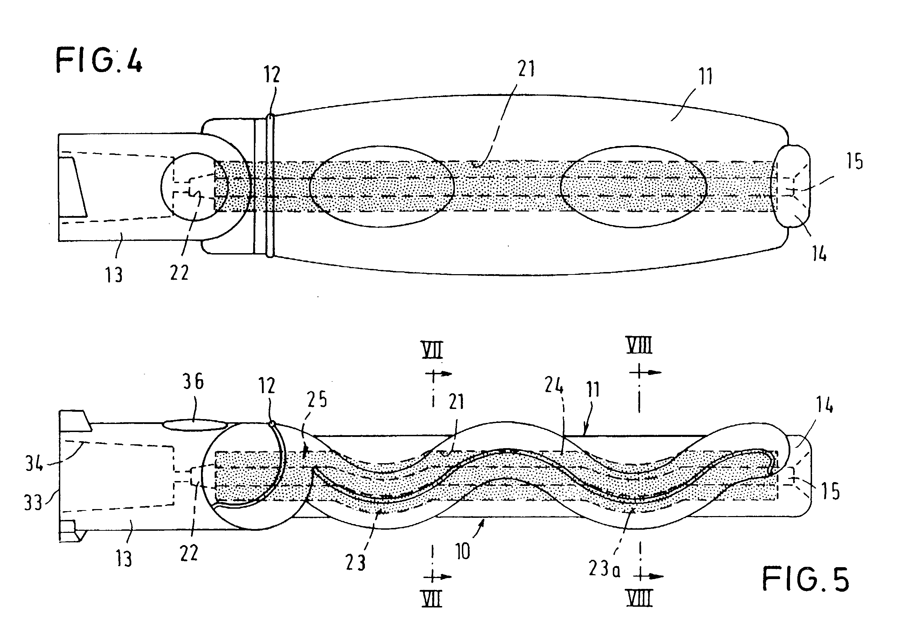

Die Backen 10,11 bilden im geschlossenen Zustand einen längslaufenden Kanal 21, der in Draufsicht gemäß Fig. 4 gerade ist und sich von einem Durchlaß 22 am Verbindungselement 13 bis zu der Stirnwand 14 erstreckt. Dieser Kanal 21, der in den Fign. 3 und 4 gerastert ist, hat in Seitenansicht gemäß Fig. 5 zwei Biegungen 23,23a. Beide Biegungen erstrecken sich in dieselbe Richtung, hier in den Backen 10 hinein. Der Kanal 21 durchläuft also in den Biegungen 23,23a jeweils ein Tal. In dem zwischen den Biegungen 23,23a liegenden Abschnitt 24 verläuft der Kanal 21 geradlinig und axial zu seinen Endbereichen. Die Biegungen 23,23a und der gerade Abschnitt 24 haben jeweils etwa die gleiche Länge. Der Kanal 21 hat kreisrunden Querschnitt. Er setzt sich zusammen aus einer halbkreisförmigen Rinne 21a im oberen Backen und einer halbkreisförmigen Rinne 21b im unteren Backen.

Der Kanal 21 enthält ein Schlauchstück 25, das sich von dem Durchlaß 22 bis zur Öffnung 15 erstreckt. Die Enden des Schlauchstücks 25 können jeweils mit dem Körper des Backen 10 verklebt sein. Das Schlauchstück 25 ist bleibender Bestandteil der Katheterkupplung.

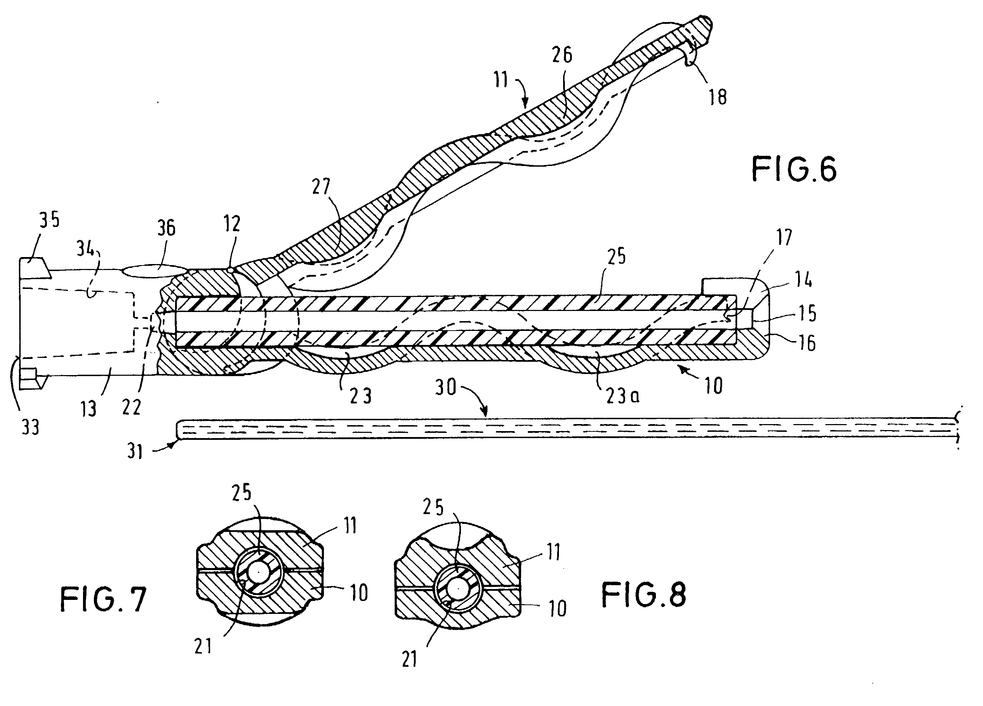

Bei geöffnetem Backen 11 nimmt das zum Backen 10 gehörende Schlauchstück 25 gemäß Fig. 6 eine gerade Form an, wobei es sich von dem Boden der beiden Biegungen 23,23a abhebt. Im Boden der Rinne 21a des oberen Backens 11 sind zwei Erhöhungen 26,27 vorgesehen. Im Boden der Rinne 21b sind entsprechende Vertiefungen 46,47 vorgesehen.

In Fig. 6 ist der geöffnete Zustand der Backen dargestellt. In diesem Zustand wird durch die Öffnung 15 der proximale Endbereich des Katheters 30 in den Schlauch 25 eingeschoben, bis die Katheterspitze 31 sich in dem Durchlaß 22 befindet.

Das Verbindungselement 13 besteht aus transparentem Kunststoff. In die Außenfläche des Verbindungselements ist eine Linse 36 eingeformt, durch die der Durchlaß 22 vergrößert betrachtet werden kann. Auf diese Weise kann leicht festgestellt werden, ob die Katheterspitze 31 sich an der korrekten Stelle befindet.

Das Verbindungsstück 13 ist ein Luer-Lock-Steckverbinder 33, der einen Innenkonus 34 und an seiner Außenseite gewindeartige Vorsprünge 35 aufweist. Der Steckverbinder 33 wird mit einem komplementären Gegensteckverbinder zusammengesteckt, um den Katheter 30, beispielsweise über ein Filter, mit einer Flüssigkeitsquelle zu verbinden.

Aus den Fign. 7 und 8 geht hervor, daß der Kanal 21 sowohl in geraden Abschnitten 24 als auch an den Stellen der Biegungen 23,23a konstanten Querschnitt gleicher Größe hat. Beim Schließen der Backen 10,11 wird das Schlauchstück 25 geringfügig komprimiert, so daß es sich fest um den Katheter 30 legt. Die Zugsicherung des Katheters wird dadurch erreicht, daß das Schlauchstück zusammen mit dem darin angeordneten Katheter in die Biegungen 23,23a des Kanals 21 eingedrückt wird.

Während bei dem dargestellten Ausführungsbeispiel die Biegungen senkrecht zu der Ebene der Backen verlaufen, können sie bei einer anderen Ausführungsformn in der Ebene der Backen, bzw. in der Teilungsebene, verlaufen.

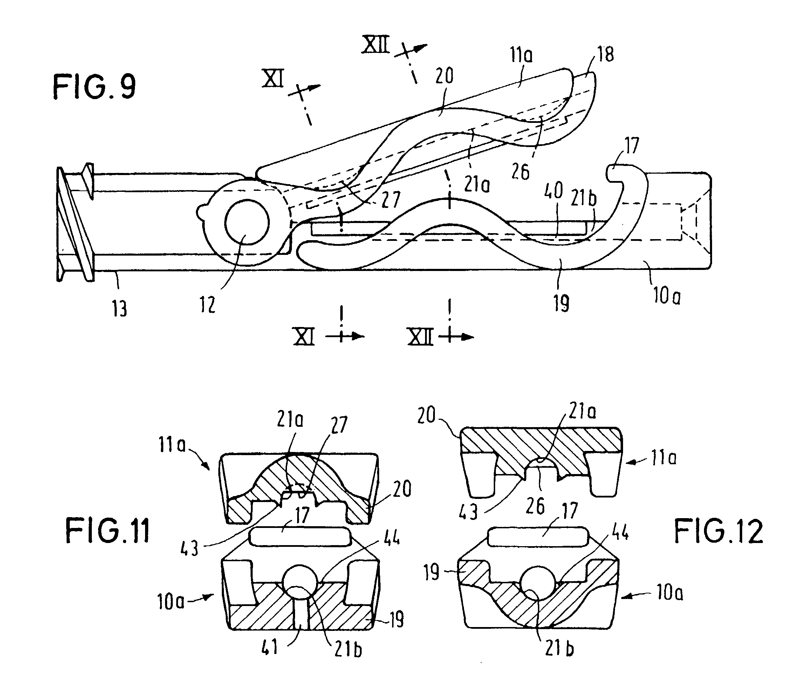

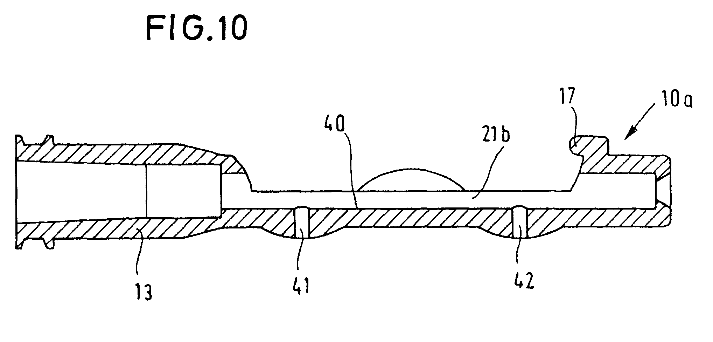

Bei dem Ausführungsbeispiel der Fign. 9-12 sind ebenfalls der untere Backen 10a und der obere Backen 11a durch ein Gelenk 12 verbunden. An den gegenüberliegenden Enden der Backen 10a und 11a befinden sich Verriegelungselemente 17,18, die bei geschlossenem oberem Backen 11a die beiden Backen relativ zueinander verriegeln. Das Schlauchstück 35 ist bei dem zweiten Ausführungsbeispiel aus Gründen der Übersichtlichkeit nicht dargestellt. Die das Schlauchstück 25 aufnehmende Rinne 21 hat einen geraden Rinnenboden 40, in dem keine Erhöhungen oder Vertiefungen vorgesehen sind. Die Rinne 21b hat generell halbkreisförmigen Querschnitt. Im Rinnenboden sind an zwei Stellen Ausweichöffnungen 41,42 vorgesehen, in die hinein das weiche Material des Schlauchstücks 25 ausweichen kann, wenn der obere Klemmbacken geschlossen wird.

An dem Rinnenboden des oberen Backens 11a sind wie bei dem ersten Ausführungsbeispiel Erhöhungen 26 und 27 vorgesehen, die durch einen geraden Abschnitt voneinander getrennt sind. Diese Erhöhungen bewirken auf dem geraden Rinnenboden 40 des unteren Backens 10a jeweils eine örtliche Quetschung des Schlauchstückes und des in dem Schlauchstück verlaufenden Katheters. Die Erhöhungen 26,27 komprimieren also das Schlauchstück 25.

Der Kanal 21 wird umfangsmäßig von den beiden gegeneinandergesetzten Rinnen 21a und 21b begrenzt. Wie die Fign. 11 und 12 zeigen, sind an der einen Rinne 21a längslaufende Ansätze in Form dreieckiger Leisten vorgesehen. An der gegenüberliegenden Rinne 21b befinden sich entsprechende Aussparungen 44, in die die Ansätze 43 bei geschlossenen Backen eintauchen. Obwohl die Trennungslinie der Backen 10a,11a diametral durch den Kanal 21 hindurchgeht, umschließt der obere Backen 10a mit den beiden längslaufenden Ansätzen 43 mehr als die Hälfte des Kanalumfangs, während der untere Backen 11a wegen der Aussparungen 44 weniger als die Hälfte des Kanalumfangs begrenzt. Durch die Ansätze 43 wird verhindert, daß das Material des (nicht dargestellten) Schlauchstücks, das sich in dem Kanal 21 befindet, in den Spalt zwischen den beiden Backen kriechen kann. Außerdem wird beim Schließen der Backen eine exakte Führung sichergestellt.

Bei dem zweiten Ausführungsbeispiel sind Erhöhungen 26,27 nur an dem oberen Backen 11a vorgesehen, so daß sich der Querschnitt des Kanals 21 an den Stellen der Erhöhungen 26,27 verringert. An diesen Stellen wird das Schlauchstück gequetscht.

## Patentansprüche

1. Katheterkupplung mit einem Schlauchstück und mit zwei Backen (10,11), die in aneinanderliegendem Zustand einen Kanal (21) mit dem Schlauchstück (25) zur Aufnahme des Endbereichs eines Katheters (30) bilden, wobei
die Backen (10,11) an einem Ende durch ein Gelenk (12) verbunden sind und am gegenüberliegenden Ende zusammengreifende Verriegelungselemente (17,18) aufweisen, **dadurch gekennzeichnet, daß** die Backen (10,11) in Längsrichtung des Schlauchstücks (25) verlaufen und die Achse des Gelenks (12) quer zu dem Schlauchstück verlauft.

2. Katheterkupplung nach Anspruch 1, **dadurch gekennzeichnet, daß** die Backen (10,11) derart ausgebildet sind, daß der Kanal (21) mindestens eine Querschnittsverengung aufweist, in deren Verlauf der Katheter (30) verformt bzw. geklemmt wird.

3. Katheterkupplung nach Anspruch 2, **dadurch gekennzeichnet, daß** der Kanal (21) mindestens zwei Querschnittsverengungen aufweist, zwischen denen sich ein längslaufender gerader Abschnitt (24) befindet.

4. Katheterkupplung nach Anspruch 1, **dadurch gekennzeichnet, daß** einer der Backen eine Rinne (21a) mit mindestens zwei hintereinanderliegenden Erhöhungen (26,27) aufweist, und der gegenüberliegende Backen (10a) eine gerade Rinne (21b) aufweist.

5. Katheterkupplung nach einem der Ansprüche 1-4, **dadurch gekennzeichnet, daß** ein Backen (10) einen Steckverbinder (33) zum Ankuppeln eines Gegensteckverbinders aufweist und den einen Endbereich des Kanals (21) ungeteilt enthält.

6. Katheterkupplung nach Anspruch 5, **dadurch gekennzeichnet, daß** angrenzend an den Steckverbinder (33) eine Linse (36) vorgesehen ist, die das Betrachten eines zwischen dem Kanal (21) und dem Steckverbinder (33) verlaufenden Durchlasses (22) ermöglicht.

7. Katheterkupplung nach einem der Ansprüche 1-6, **dadurch gekennzeichnet, daß** der Kanal (21) aus zwei Rinnen (21a,21b) besteht, von denen die eine Rinne durch längslaufende Ansätze (43) begrenzt ist, welche in entsprechende Aussparungen (44) der anderen Rinne (21b) eingreifen.

## Claims

1. A catheter coupling with a hose piece and two jaws (10, 11) that, when closed, form a channel (21) with said hose piece (25) for receiving the end portion of a catheter (30), wherein the jaws (10, 11) are connected at one end by a joint (12) and have interlocking lock elements (17, 18) at the opposite end,
**characterized in that**
the jaws (10, 11) extend in the longitudinal direction of the hose piece (25) and the axis of the joint (12) extends transversely to the hose piece.

2. The catheter coupling of claim 1, wherein the jaws (10, 11) are formed such that the channel (21) has at least one constriction of the cross section where the catheter (30) is deformed or clamped.

3. The catheter coupling of claim 2, wherein the channel (21)has at least two constrictions of the cross section between which extends a longitudinal straight section (24).

4. The catheter coupling of claim 1, wherein one of the jaws has a groove (21a) with at least two successive raised portions (26, 27), and the opposite jaw (10a) has a straight groove (21b).

5. The catheter coupling of one of claims 1-4, wherein one jaw (10) has a plug connector (33) for coupling a counter plug connector thereto, and the jaw comprises the one end portion of the channel (21) as a whole.

6. The catheter coupling of claim 5, wherein a lens (36) is provided adjacent the plug connector (33), which allows observing a passage (22) extending between the channel (21) and the plug connector (33).

7. The catheter coupling of one of claims 1-6, wherein the channel (21) consists of two grooves (21a, 21b) one of which is defined by longitudinally extending projections (43) engaging into corresponding recesses (44) in the other groove (21b).

## Revendications

1. Dispositif de raccordement pour cathéter comportant un morceau de tuyau souple ainsi que deux mâchoires (10, 11) qui, dans l'état où elles sont appliquées l'une contre l'autre, forment un canal (21) avec le morceau de tuyau souple (25), pour recevoir la partie d'extrémité d'un cathéter (30), les mâchoires (10, 11) étant, à une extrémité, reliées par une articulation (12) et présentant, à l'extrémité opposée, des éléments de verrouillage (17, 18) s'accrochant ensemble, **caractérisé en ce que** les mâchoires (10, 11) s'étendent dans la direction longitudinale du morceau de tuyau souple (25) et l'axe de l'articulation (12) s'étend transversalement au morceau de tuyau souple.

2. Dispositif de raccordement pour cathéter selon la revendication 1, **caractérisé en ce que** les mâchoires (10, 11) sont dotées d'une configuration telle, que le canal (21) présente au moins un resserrement de sa section transversale, sur l'étendue duquel le cathéter (30) est déformé ou coincé.

3. Dispositif de raccordement pour cathéter selon la revendication 2, **caractérisé en ce que** le canal (21) présente au moins deux resserrements de sa section transversale, entre lesquels se trouve un tronçon rectiligne (24) d'extension longitudinale.

4. Dispositif de raccordement pour cathéter selon la revendication 1, **caractérisé en ce que** l'une des mâchoires présente une rainure (21a) comportant au moins deux proéminences (26, 27) situées l'une à la suite de l'autre, et la mâchoire opposée (10a) présente une rainure rectiligne (21b).

5. Dispositif de raccordement pour cathéter selon l'une des revendications 1 à 4, **caractérisé en ce qu'**une mâchoire (10) comporte un connecteur à enfichage (33) destiné à l'accouplement d'un connecteur à enfichage complémentaire et contient, d'une façon non divisée, l'une des parties d'extrémité du canal (21).

6. Dispositif de raccordement pour cathéter selon la revendication 5, **caractérisé en ce que** dans une position contiguë au connecteur à enfichage (33), il est prévu une lentille (36), qui permet d'observer un passage (22) s'étendant entre le canal (21) et le connecteur à enfichage (33).

7. Dispositif de raccordement pour cathéter selon l'une des revendications 1 à 6, **caractérisé en ce que** le canal (21) est constitué de deux rainures (21a, 21b), dont l'une est délimitée par des appendices (43) d'extension longitudinale, qui s'engagent dans des évidements correspondants (44) de l'autre rainure (21b).
